(19) Europäisches Patentamt
European Patent Office
Office européen des brevets

(11) Numéro de publication: **0 213 976 B1**

# (12) FASCICULE DE BREVET EUROPEEN

(45) Date de publication du fascicule du brevet: **22.08.90**

(21) Numéro de dépôt: **86401417.0**

(22) Date de dépôt: **27.06.86**

(51) Int. Cl.[5]: **C07C 51/60**, C07C 51/58, C07C 53/42, C07C 53/50, C07C 53/46, C07C 63/10

(54) **Nouveau procédé de préparation de chlorures d'acides par phosgénation des acides et catalyseurs pour ce procédé.**

(30) Priorité: **23.07.85 FR 8511248**

(43) Date de publication de la demande:
**11.03.87 Bulletin 87/11**

(45) Mention de la délivrance du brevet:
**22.08.90 Bulletin 90/34**

(84) Etats contractants désignés:
**AT BE CH DE FR GB IT LI NL SE**

(56) Documents cités:
**EP-A- 0 027 941**
**HU-A- 160 740**

(73) Titulaire: **SOCIETE NATIONALE DES POUDRES ET EXPLOSIFS, 12, quai Henri IV, F-75181 Paris Cédex 04(FR)**

(72) Inventeur: **Senet, Jean-Pierre, 79, rue de la Gare Herbeauvilliers-Buthiers, F-77760 La Chapelle-La Reine(FR)**
Inventeur: **Gauthier, Patricia, Chemin des Acacias, F-91590 Cerny(FR)**
Inventeur: **Malfroot, Thierry, 17, Chemin des Jardins, F-91100 Saintry-sur-Seine(FR)**
Inventeur: **Wolf, Patrick, 2 rue Gay-Lussac, F-91610 Ballancourt(FR)**

EP 0 213 976 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

L'invention a pour objet un nouveau procédé de préparation de chlorures d'acides carboxyliques.

Il est connu de préparer les chlorures d'acides carboxyliques par phosgénation de l'acide carboxylique correspondant. La réaction est la suivante :

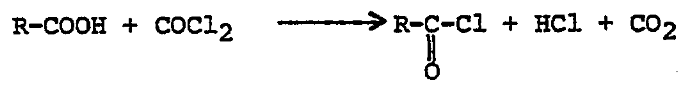

Mais en l'absence de catalyseur la réaction n'a lieu qu'à haute température (brevet FR 732 078) et dans certains cas sous une forte pression au moins égale à 10 atmosphères (brevet US 2 657 233).

L'emploi d'un catalyseur est donc indispensable pour obtenir des chlorures d'acides dans des conditions économiquement acceptables.

Le charbon actif en poudre ou de préférence en granulés est très souvent utilisé. Malheureusement, il est difficile à recycler car il perd son activité et un grand nombre de particules de charbon très fines restent en suspension dans le chlorure d'acide. Des produits secondaires insaturés ou chlorés peuvent se former, en particulier lors de la préparation des chlorures d'acides aliphatiques, provoqués par la décomposition partielle du phosgène en monooxyde de carbone et en chlore (brevet FR 839 231).

D'autres catalyseurs ont également été proposés :

- les amines tertiaires et leurs chlorhydrates (brevets FR 864 515, 2 212 319),
- les sels d'ammonium et de phosphonium quaternaires, les sels de sulfonium tertiaires (brevet GB 1 159 266),
- les amides (brevet US 3 149 155),
- les imidazoles et leurs dérivés (brevet US 3 547 960),
- les guanidines pentasubstituées (brevet HU n° 160 740),
- les urées tétrasubstituées et les phosphoramides (brevet FR 1 226 245).

Cependant tous les procédés utilisant ces catalyseurs présentent les inconvénients suivants à des degrés divers.

La quantité de catalyseur nécessaire est importante et est le plus souvent supérieure à 1 % en mole par rapport à l'acide. Il est par conséquent obligatoire de distiller le chlorure d'acide pour en éliminer les restes de catalyseur. Or cette distillation est particulièrement difficile dans le cas des chlorures d'acides peu volatils.

Les rendements sont variables et pas toujours suffisants. Il se forme des sous-produits très gênants tels que les chlorures de carbamyles et/ou des résidus goudronneux, ce qui rend délicat la mise en oeuvre des procédés et la purification des chlorures d'acides.

Il était par conséquent très important de trouver un nouveau catalyseur de préparation des chlorures d'acides qui permette de simplifier les différentes opérations et de diminuer la quantité de produits secondaires.

La présente invention a pour objet un procédé de préparation des chlorures d'acides par phosgénation des acides carboxyliques en présence d'un nouveau catalyseur.

Le catalyseur selon l'invention est choisi dans le groupe des sels de guanidinium hexasubstitués ou de leurs complexes avec les acides halohydriques, représentés par les formules :

(I) 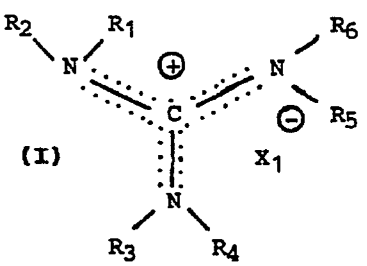

(II) 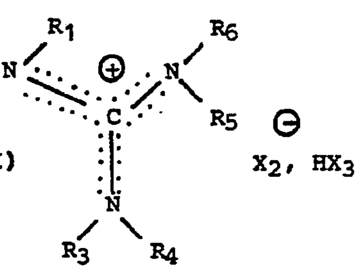

dans lesquelles $R_1$, $R_2$, $R_3$, $R_4$, $R_5$ et $R_6$ identiques ou différents représentent :

- des radicaux aliphatiques linéaires ou ramifiés, substitués ou non, saturés ou insaturés, l'insaturation n'étant pas située sur le carbone voisin de l'atome d'azote,
- des radicaux cycloaliphatiques substitués ou non, saturés ou non, l'insaturation n'étant pas située sur le carbone voisin de l'atome d'azote,
- des radicaux araliphatiques substitués ou non,
- des radicaux aromatiques substitués ou non,
- ou forment avec l'atome d'azote auquel ils sont fixés et avec l'autre radical fixé sur le même atome d'azote, un hétérocycle substitué ou non, saturé ou non, l'insaturation n'étant pas située sur le carbone voisin de l'atome d'azote,

- ou forment avec l'atome d'azote auquel ils sont fixés, le carbone central, un autre atome d'azote et un radical fixé sur cet atome d'azote, un hétérocycle substitué ou non, saturé ou non, l'insaturation n'étant pas située sur un carbone voisin des atomes d'azote,

$X_1$ représente un anion choisi dans le groupe comprenant $Cl^{\ominus}$, $Br^{\ominus}$, $I^{\ominus}$, $BF_4^{\ominus}$, $CN^{\ominus}$, $ClO_4^{\ominus}$, $NO_3^{\ominus}$, $NO_2^{\ominus}$, $OCN^{\ominus}$, $CH_3SO_4^{\ominus}$, $HSO_4^{\ominus}$ et

$X_2$ et $X_3$ identiques ou différents représentent des atomes de chlore ou de brome.

Le cation des formules précédentes peut également s'écrire sous les formes limites suivantes :

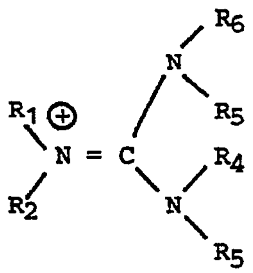
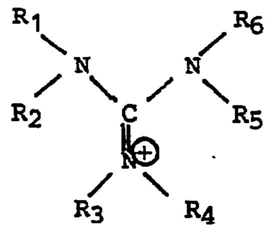
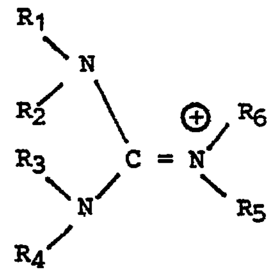

Les catalyseurs de la présente invention sont particulièrement intéressants parce qu'on a trouvé qu'ils catalysent également la décomposition des anhydrides d'acides formés comme sous-produits, dans la réaction principale.

Un autre avantage des catalyseurs de l'invention est qu'ils peuvent être utilisés en quantités nettement inférieures aux quantités généralement employées dans les autres procédés.

Ces quantités sont comprises entre $10^{-4}$ et $5x10^{-3}$ équivalent par équivalent d'acide et de préférence entre $10^{-4}$ et $10^{-3}$ équivalent.

Une quantité plus importante peut également être mise en oeuvre mais cela présente rarement un intérêt.

Les chlorures d'acide obtenus ne contiennent ainsi pratiquement pas de catalyseur en tant qu'impureté.

Pour certaines de leurs utilisations il ne sera même pas nécessaire de les distiller et une opération qui pourrait être difficile sera ainsi supprimée.

Lorsque par contre une distillation sera effectuée il sera très facile de recycler le catalyseur à partir du résidu de distillation ce qui est également très avantageux. De plus les nouveaux catalyseurs selon l'invention demeurent actifs même après plusieurs opérations successives de phosgénation.

Les sels de guanidinium hexasubstitués utilisés sont de préférence les halogénures et en particulier les chlorures de guanidinium hexasubstitués, complexés ou non par un acide halohydrique, de préférence par l'acide chlorhydrique.

Les radicaux $R_1$ à $R_6$ précédemment définis peuvent représenter par exemple des radicaux aliphatiques saturés avec de 1 à 12 atomes de carbone, de préférence avec 1 à 4 atomes de carbone ou insaturés avec de 3 à 12 atomes de carbone, de préférence avec de 3 à 8 atomes de carbone, des radicaux cycloaliphatiques saturés ou insaturés avec de 5 à 6 atomes de carbone, des radicaux araliphatiques avec de 7 à 12 atomes de carbone, en particulier des radicaux benzyle, des radicaux phényle.

$R_1$ et $R_2$ et/ou $R_3$ et $R_4$ et/ou $R_5$ et $R_6$ peuvent représenter avec l'atome d'azote auquel ils sont fixés par exemple un hétérocycle à 5 à 6 chaînons qui peut contenir un autre hétéroatome, comme l'oxygène, tel que par exemple le cycle de la morpholine.

$R_1$ et $R_6$ et/ou $R_5$ et $R_4$ et/ou $R_2$ et $R_3$ peuvent former avec le groupe N - C - N un hétérocycle avec de 5 à 6 chaînons, tel qu'en particulier le cycle

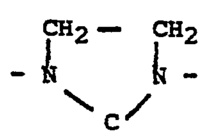

Les substituants des radicaux $R_1$ à $R_6$ sont des groupes inertes dans les conditions de réaction, choisis par exemple parmi les atomes d'halogènes, les groupes alkyles, alkoxy, aryloxy et nitro.

De préférence les radicaux $R_1$ à $R_6$ représentent les radicaux méthyle, éthyle, n-propyle, n-butyle, isopropyle, isobutyle, allyle, cyclohexyle, phényle, chloro-4 phényle, benzyle.

Les catalyseurs selon l'invention peuvent se préparer de façon connue selon plusieurs procédés.

Par exemple, ils peuvent être préparés à partir d'une urée ou thiourée qui est phosgénée pour obtenir le chlorure de chloroformamidinium (Chem Ber. 97 p.1232 (1964)) que l'on met ensuite à réagir avec une amine (Synthesis 1983 (11) 904-905) ou bien par réaction d'une thiourée avec un chlorure de carbamyle (Liebigs Ann. Chem, 1984, 108 - 126) ou encore à partir de pentaguanidines que l'on fait réagir sur un dérivé halogéné (Houben Weyl VIII p.100 et 186, 1952), les pentaguanidines étant elles-mêmes obtenues par réaction d'un isocyanatodihalogénure sur une amine secondaire (brevet FR 1 453 438).

Les complexes de sels de guanidinium hexasubstitués peuvent être préparés par addition de l'acide halohydrique sur les sels de guanidinium hexasubstitués.

Le complexe du chlorure de guanidinium hexasubstitué avec l'acide chlorhydrique est préparé en particulier par réaction du chlorure de chloroformamidinium sur une amine secondaire suivie, sans séparation des produits obtenus, par une phosgénation :

Les réactions sont les suivantes :

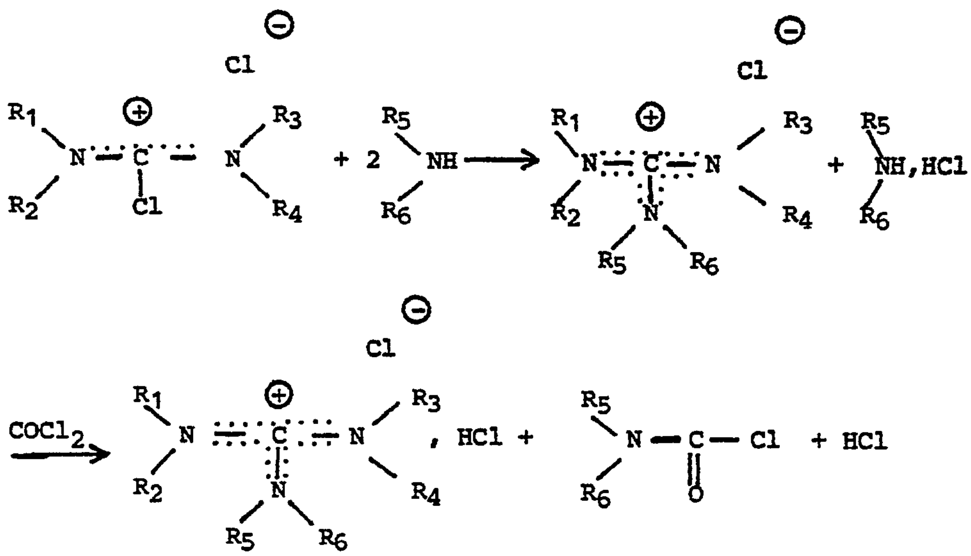


Ce procédé est particulièrement intéressant car il n'est pas nécessaire d'éliminer par un excès d'une base le chlorhydrate d'amine formé dans la première étape puisqu'il est transformé dans la deuxième étape en chlorure de carbamyle qui peut être séparé facilement par distillation.

Les chlorures d'acide peuvent être obtenus selon l'invention à partir de la plupart des acides carboxyliques.

A titre d'exemple on peut citer les acides carboxyliques de formule R $(COOH)_n$ dans laquelle n = 1 ou 2 ou 3 et R représente

- un radical aliphatique linéaire ou ramifié, substitué ou non, saturé ou non, avec jusqu'à 30 atomes de carbone,
- un radical cycloaliphatique en $C_3$ à $C_6$ substitué ou non, saturé ou non,
- un radical araliphatique substitué ou non,
- un radical aromatique substitué ou non,
- un radical hétérocyclique.

Comme substituants du radical R on peut citer par exemple les atomes d'halogènes en particulier de chlore, fluor et brome, des groupes alkyles ou halogénoalkyles, alkoxy, phénoxy et nitro.

Les acides carboxyliques préférés dans le cadre de la présente invention sont choisis dans le groupe constitué par l'acide acétique, l'acide propionique, l'acide butyrique, l'acide éthyl-2 hexanoïque, l'acide nonanoïque, l'acide stéarique, l'acide benzoïque, l'acide laurique, l'acide palmitique, l'acidde undécylénique, l'acide acrylique, l'acide méthacrylique.

La réaction de phosgénation des acides avec les catalyseurs de l'invention a lieu de préférence sans solvant.

Lorsque les acides ont un faible point d'ébullition ou un point de fusion élevé, par exemple supérieur à 100°C, on peut utiliser un solvant ou un mélange de solvants inertes vis à vis du phosgène choisis par exemple parmi les hydrocarbures aliphatiques chlorés, comme le dichloroéthane, les hydrocarbures aromatiques comme le toluène, le xylène, le chlorobenzène ou le dichlorobenzène.

Le phosgène est de préférence introduit gazeux dans le milieu réactionnel qui est porté et maintenu à la temperature convenable. On préfère le plus souvent utiliser un léger excès de phosgène généralement compris entre 5 et 25%.

Il est également possible d'opérer en continu en introduisant simultanément le phosgène et l'acide dans le réacteur et en séparant le chlorure d'acide formé en continu.

La réaction est généralement effectuée à une température comprise entre 80° et 160°C et de préférence entre 100 et 140°C.

Grâce au procédé de l'invention on obtient des chlorures d'acides pratiquement exempts d'impuretés. Les opérations de purification sont simplifiées. Le catalyseur peut être facilement recyclé.

Les chlorures d'acide sont des composés connus qui ont de très nombreuses applications. Ils sont particulièrement utiles pour la préparation de peresters et de peroxydes, initiateurs de polymérisation radicalaire, et de produits pesticides.

Les exemples qui suivent illustrent l'invention sans toutefois la limiter.

Exemple 1

Préparation du complexe chlorure d'hexa n-butylguanidinium, acide chlorhydrique

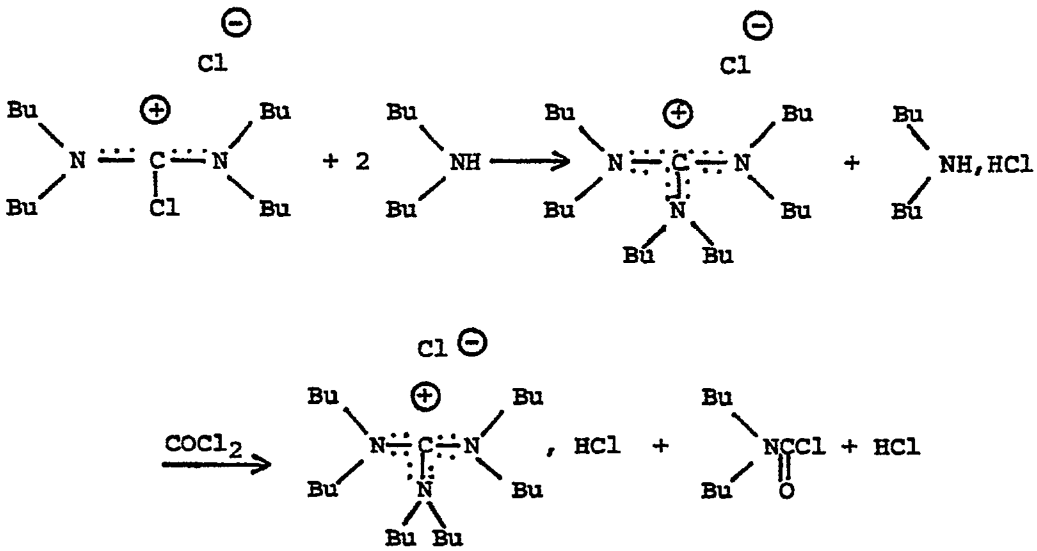


Dans un réacteur équipé, on introduit 0,06 mole de di-n-butylamine en solution dans 10 ml de toluène. On additionne ensuite 0,03 mole de chlorure de tétrabutylchloroformamidinium en solution dans 25 ml de toluène en laissant la température évoluer entre 30° et 60°C.

On agite le milieu réactionnel une demi-heure à la température voisine de 20°C puis on introduit à une température voisine de 20°C, 0,04 mole de phosgène gazeux.

On porte à 110°C puis on agite pendant 2 heures.

Après dégazage et élimination du toluène par évaporation sous vide, on reprend le résidu à l'éther. On obtient 10,5 g (rendement 75 %) du complexe attendu qui a précipité sous forme de cristaux blancs.

Point de fusion : 90°C

Analyse élémentaire :

| | C | H | N | Cl |
|---|---|---|---|---|
| Calculé | 64.1 | 11,75 | 8,88 | 15,17 |
| Trouvé | 63,42 | 11,48 | 8,08 | 15,31 |

Exemples 2 à 17 :

Préparation des chlorures d'acides.

On prépare différents chlorures d'acides selon le mode opératoire suivant :

Dans un réacteur équipé d'une agitation, d'un thermomètre, d'un tube plongeant et d'un réfrigérant à reflux refroidi à -70°C, on introduit 1 mole d'acide carboxylique, une quantité variable de catalyseur et éventuellement le solvant.

On porte le mélange réactionnel à la température voulue et on introduit lentement 1,2 mole environ (119 g) de phosgène gazeux, tout en maintenant le température.

L'introduction du phosgène terminée, on laisse sous agitation à latempérature de phosgénation jusqu'à disparition totale de l'acide résiduel et de l'anhydride formé (1 heure en moyenne).

On élimine ensuite le phosgène en excès par dégazage à l'azote ou sous pression réduite.

Le chlorure d'acide obtenu est, s'il y a lieu, purifié par distillation.

Le dosage potentiométrique des chlorures d'acides est effectué endosant le chlorohydrate d'amine formé par réaction de la m-chloroaniline sur le chlorure d'acide selon la méthode Siggia Procédure B.226-1979.

Les acides, les catalyseurs et les conditions opératoires utilisées ainsi que les résultats obtenus sont rassemblés dans le tableau suivant.

| EX N° | ACIDE | CATALYSEUR | % molaire catalyseur/acide | Durée totale de phosgénation (h) | Température °C | Rendement en chlorure % | Pureté * % |
|---|---|---|---|---|---|---|---|
| 2 | Butyrique | 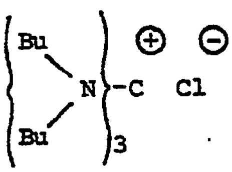 | 0,1 | 6 | 125-102 | distillé 90 | 98,7 |
| 3 | Ethyl-2 hexanoïque | " | 0,1 | 1 | 130-140 | distillé 94 | 99 |
| 4 | " | " | 0,1 | 6 | 80-85 | distillé 93 | 99,5 |
| 5 | " | " | 0,02 | 6 | 120-125 | distillé 94 | 99,7 |
| 6 | " | 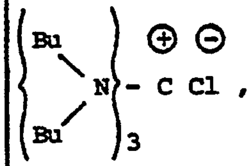 , HCl | 0,02 | 6 | 120-125 | distillé 93 | 99,9 |

* Taux de chlore par analyse potentiométrique (%)

| EX N° | ACIDE | CATALYSEUR | % molaire catalyseur/acide | Durée totale de phosgénation (h) | Température °C | Rendement en chlorure % | Pureté * % |
|---|---|---|---|---|---|---|---|
| 7 | " | 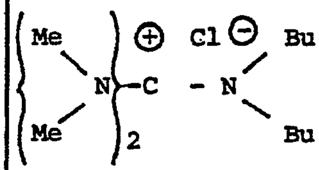 | 0,05 | 3h45 | 120-125 | distillé 93 | 99,9 |
| 8 | Nonanoïque | 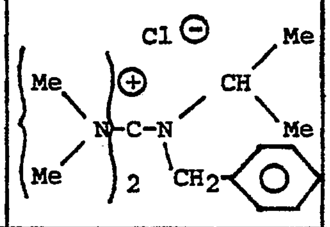 | 0,05 | 4h30 | 120-125 | distillé 90 | 100 |
| 9 | Stéarique | 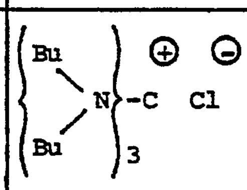 | 0,05 | 2h30 | 130-140 | brut 100 | 99,3 |
| 10 | " | " | 0,02 | 4 | 120-125 | brut 100 | 100 |
| 11 | " | 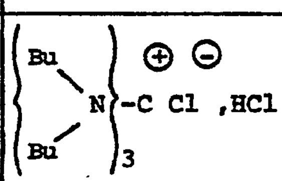 | 0,02 | 4 | 120-125 | brut 100 | 100 |
| 12 | Benzoïque | 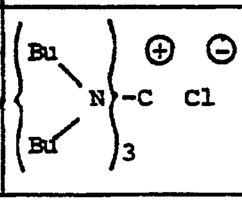 | 0,1 | 6 | 120-125 | distillé 93 | 100 |

| EX N° | ACIDE | CATALYSEUR | % molaire catalyseur/acide | Durée totale de phosgénation (h) | Température °C | Rendement en chlorure % | Pureté * % |
|---|---|---|---|---|---|---|---|
| 13 | Laurique | $[(Bu)_2N]_3C^{\oplus}\ Cl^{\ominus}$ ,HCl | 0,02 | 7h | 115-120 | distillé 90 | 99 |
| 14 | Palmitique | " | 0,02 | 6h | 115-120 | brut 100 | 98 |
| 15 | Undécylénique | " | 0,1 | 4h | 115-120 | distillé 83 | 98 |
| 16 | Propionique | $[(CH_3)_2N]_3\ C^{\oplus}\ Cl^{\ominus}$ | 0,2 | 7h | 80-85 | distillé 94 | 97 |
| 17 | Acrylique | $[(CH_3)_2N]_3\ C^{\oplus}\ Cl^{\ominus}$ HCl | 0,5 | 7h | 115-150 | distillé 74 | 99 |

## Revendications

1°) Procédé de préparation de chlorures d'acides carboxyliques par réaction du phosgène sur un acide carboxylique en présence d'un catalyseur caractérisé en ce que le dit catalyseur est choisi dans le groupe constitué par les sels de guanidinium hexasubstitués et leurs complexes de formules :

(I) 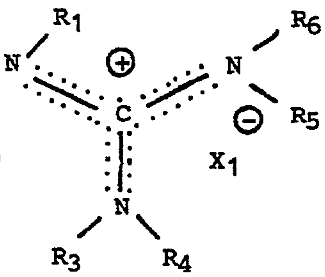

(II) 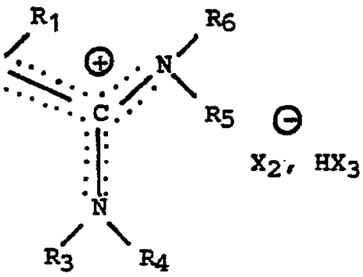

dans lesquelles $R_1$, $R_2$, $R_3$, $R_4$, $R_5$ et $R_6$ identiques ou différents représentent :
- des radicaux aliphatiques linéaires ou ramifiés, substitués ou non, saturés ou insaturés, l'insaturation n'étant pas située sur le carbone voisin de l'atome d'azote,
- des radicaux cycloaliphatiques substitués ou non, saturés ou non, l'insaturation n'étant pas située sur le carbone voisin de l'atome d'azote,
- des radicaux araliphatiques substitués ou non,
- des radicaux aromatiques substitués ou non,
- ou forment avec l'atome d'azote auquel ils sont fixés et avec l'autre radical fixé sur le même atome d'azote, un hétérocycle substitué ou non, saturé ou non, l'insaturation n'étant pas située sur le carbone voisin de l'atome d'azote,
- ou forment avec l'atome auquel ils sont fixés, le carbone central, un autre atome d'azote et un radical fixé sur cet atome d'azote, un hétérocycle substitué ou non, saturé ou non, l'insaturation n'étant pas situé sur un carbone voisin des atomes d'azote,

$X_1$ représente un anion choisi dans le groupe comprenant $Cl^{\ominus}$, $Br^{\ominus}$, $I^{\ominus}$, $BF_4^{\ominus}$, $CN^{\ominus}$, $ClO_4^{\ominus}$, $NO_3^{\ominus}$, $NO_2^{\ominus}$, $OCN^{\ominus}$, $CH_3SO_4^{\ominus}$, $HSO_4^{\ominus}$ et

$X_2$ et $X_3$ identiques ou différents représentent des atomes de chlore ou de brome.

2°) Procédé selon la revendication 1 caractérisé en ce que les radicaux $R_1$ à $R_6$ sont des radicaux aliphatiques saturés avec de 1 à 12 atomes de carbone ou insaturés avec de 3 à 12 atomes de carbone, des radicaux cycloaliphatiques saturés ou insaturés avec de 5 à 6 atomes de carbone, des radicaux araliphatiques avec de 7 à 12 atomes de carbone, des radicaux phényles, ou forment avec le radical situé sur le même atome d'azote et celui-ci un hétérocycle à 5 ou 6 chaînons qui peut contenir un autre hétéroatome ou forment avec le radical situé sur un autre atome d'azote et le groupe -N-C-N- un hétérocycle à 5 ou 6 chaînons.

3°) Procédé selon l'une quelconque des revendications précédentes caractérisé en ce que $R_1$ à $R_6$ sont des radicaux aliphatiques saturés avec de 1 à 4 atomes de carbone ou insaturés avec de 3 à 8 atomes de carbone, des radicaux cylcoaliphatiques avec de 5 à 6 atomes de carbone, des radicaux benzyle.

4°) Procédé selon l'une quelconque des revendications précédentes caractérisé en ce que les substituants des radicaux $R_1$ à $R_6$ sont choisis dans le groupe qui comprend les atomes d'halogène, les groupes alkyles, alkoxy, aryloxy et nitro.

5°) Procédé selon l'une quelconque des revendications précédentes caractérisé en ce qui $X_1$ est un atome de chlore ou de brome et $X_2$ et $X_3$ représentent des atomes de chlore.

6°) Procédé selon l'une quelconque des revendications précédentes caractérisé en ce qu'on utilise le catalyseur en quantité comprise entre $10^{-4}$ et $5x10^{-3}$ équivalent par équivalent d'acide, de préférence entre $10^{-4}$ et $10^{-3}$ équivalent.

7°) Procédé selon l'une quelconque des revendications précédentes caractérisé en ce qu'on utilise un excès de phosgène compris entre 5 et 25 %.

8°) Procédé selon l'une quelconque des revendications précédentes caractérisé en ce que la température de la réaction est comprise entre 80° et 160°C, de préférence entre 100° et 140°C.

9°) Procédé selon l'une quelconque des revendications précédentes caractérisé en ce qu'on effectue la réaction dans un solvant ou un mélange de solvants inertes vis-à-vis du phosgène choisis parmi les hydrocarbures aliphatiques chlorés et les hydrocarbures aromatiques.

10°) Procédé selon l'une quelconque des revendications précédentes caractérisé en ce que l'acide carboxylique est un acide de formule $R(COOH)_n$ dans laquelle n = 1,2 ou 3 et R représente - un radical aliphatique linéaire ou ramifié, substitué ou non, saturé ou non, avec jusqu'à 30 atomes de carbone,
- un radical cycloaliphatique en $C_3$ à $C_6$ substitué ou non,
- un radical araliphatique substitué ou non,
- un radical aromatique substitué ou non,
- un radical hétérocyclique.

11°) Procédé selon l'une quelconque des revendications précédentes caractérisé en ce que les substituants du radical R sont choisis dans le groupe qui comprend les atomes d'halogène, les groupes alkyles, halogénoalkyles, alkoxy, phénoxy et nitro.

12°) Procédé selon l'une quelconque des revendications précédentes caractérisée en ce que le procédé est réalisé en continu par introduction simultanée d'acide et de phosgène.

13°) Procédé de préparation du complexe de formule II dans laquelle $X_2$ et $X_3$ sont des atomes de chlore caractérisé en ce que les produits obtenus par réaction du chlorure de chloroformamidinium avec une amine secondaire sont mis à réagir avec du phosgène.

## Claims

1. Process for the preparation of carboxylic acid chlorides by the reaction of phosphene with a carboxylic acid in the presence of a catalyst, characterized in that the said catalyst is chosen from the group consisting of hexasubstituted guanidinium salts and their complexes of formulae

(I) 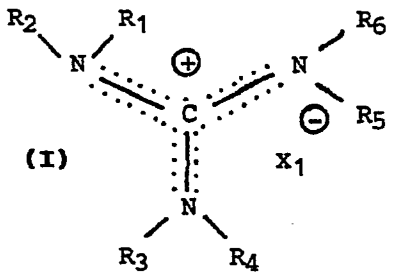

(II) 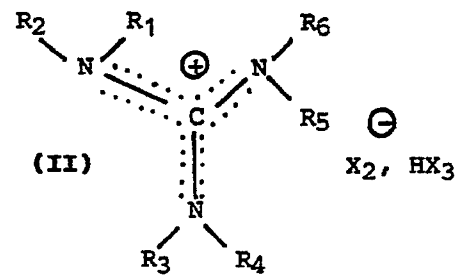

in which $R_1$, $R_2$, $R_3$, $R_4$, $R_5$ and $R_6$, which are identical or different, denote:

– saturated or unsaturated, substituted or unsubstituted, linear or branched aliphatic radicals, the unsaturation not being situated on the carbon adjoining the nitrogen atom,

– saturated or unsaturated, substituted or unsubstituted cycloaliphatic radicals, the unsaturation not being situated on the carbon adjoining the nitrogen atoms,

– substituted or unsubstituted araliphatic radicals,

– substituted or unsubstituted aromatic radicals,

– or form, with the nitrogen atom to which they are attached and with the other radical attached to the same nitrogen atom, a saturated or unsaturated, substituted or unsubstituted heterocyclic ring, the unsaturation not being situated on the carbon adjoining the nitrogen atom,

– or form, with the nitrogen atom to which they are attached, the central carbon, another nitrogen atom and a radical attached to this nitrogen atom, a saturated or unsaturated, substituted or unsubstituted heterocyclic ring, the unsaturation not being situated on a carbon adjoining the nitrogen atoms,

$X_1$ denotes an anion chosen from the group comprising $Cl^\ominus$, $BR^\ominus$, $I^\ominus$, $BF_4^\ominus$, $CN^\ominus$, $ClO_4^\ominus$, $NO_3^\ominus$, $NO_2^\ominus$, $OCN^\ominus$, $CH_3SO_4^\ominus$, $HSO_4^\ominus$ and

$X_2$ and $X_3$, which are identical or different, denote chlorine or bromine atoms.

2. Process according to Claim 1, characterized in that the radicals $R_1$ to $R_6$ are saturated aliphatic radicals, containing from 1 to 12 carbon atoms or unsaturated aliphatic radicals containing from 3 to 12 carbon atoms, saturated or unsaturated cycloaliphatic radicals containing from 5 to 6 carbon atoms, araliphatic radicals containing from 7 to 12 carbon atoms, or phenyl radicals, or form, with the radical situated on the same nitrogen atom and the latter a 5- or 6-membered heterocyclic ring which may contain another heteroatom, or form, with the radical situated on another nitrogen atom and the –N–C–C-group, a 5- or 6-membered heterocyclic ring.

3. Process according to either of the preceding claims, characterized in that $R_1$ to $R_6$ are saturated aliphatic radicals containing from 1 to 4 carbon atoms or unsaturated aliphatic radicals containing from 3 to 8 carbon atoms, cycloaliphatic radicals containing from 5 to 6 carbon atoms, or benzyl radicals.

4. Process according to any one of the preceding claims, characterized in that the substituents in the radicals $R_1$ to $R_6$ are chosen from the group which comprises halogen atoms and alkyl, alkoxy, aryloxy and nitro groups.

5. Process according to one of the preceding claims, characterized in that $X_1$ is a chlorine or bromine atom and $X_2$ and $X_3$ denote chlorine atoms.

6. Process according to any one of the preceding claims, characterized in that the catalyst is used in a quantity of between $10^{-4}$ and $5 \times 10^{-3}$ equivalent per equivalent of acid, preferably between $10^{-4}$ and $10^{-3}$ equivalent.

7. Process according to any one of the preceding claims, characterized in that an excess of phosgene of between 5 and 25% is used.

8. Process acocrding to any one of the preceding claims, characterized in that the reaction temperature is between 80° and 160°C, preferably between 100° and 140°C.

9. Process according to any one of the preceding claims, characterized in that the reaction is carried out in a solvent or a mixture of solvents which are inert towards phosgene and are chosen from chlorinated aliphatic hydrocarbons and aromatic hydrocarbons.

10. Process according to any one of the preceding claims, characterized in that the carboxylic acid is an acid of formula $R(COOH)_n$ in which n = 1, 2 or 3 and R denotes

- a saturated or unsaturated, substituted or unsubstituted, linear or branched aliphatic radical containing up to 30 carbon atoms,
- a substituted or unsubstituted $C_3$–$C_6$ cycloaliphatic radical,
- a substituted ot unsubstituted araliphatic radical,
- a substituted or unsubstituted aromatic radical, or
- a heterocyclic radical.

11. Process according to any one of the preceding claims, characterized in that the substituents in the radical R are chosen from the group which comprises halogen atoms and alkyl, haloalkyl, alkoxy, phenoxy and nitro groups.

12. Process according to any one of the preceding claims, characterized in that the process is carried out continuously by simultaneous introduction of acid and phosgene.

13. Process for the preparation of the complex of formula II in which $X_2$ and $X_3$ are chlorine atoms, characterized in that the products obtained by the reaction of chloroformamidinium chloride with a secondary amine are reacted with phosgene.

**Patentansprüche**

1. Verfahren zur Herstellung von Carbonsäurechloriden durch Umsetzung von Phosgen mit einer Carbonsäure in Gegenwart eines Katalysators, dadurch gekennzeichnet, daß der Katalysator unter hexasubstituierten Guanidiniumsalzen und ihren Komplexen der Formeln I und II ausgewählt wird,

(I) 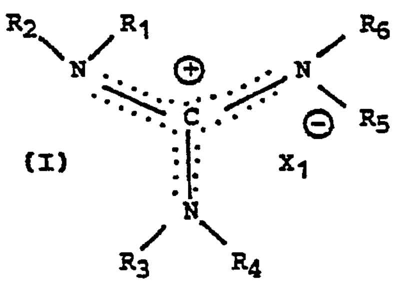

(II) 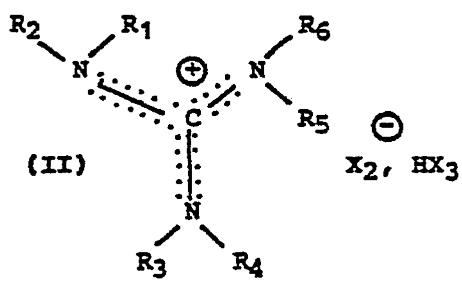

,

worin bedeuten:

$R_1$, $R_2$, $R_4$, $R_5$ und $R_6$ gleichzeitig oder unabhängig
- geradkettige oder verzweigte, gesättigte oder ungesättigte und ggfs. substituierte aliphatische Gruppen, wobei die Ungesättigtheit nicht an dem dem Stickstoffatom benachbarten C-Atom vorliegt,
- gesättigte oder ungesättigte und ggfs. substituierte cycloaliphatische Gruppen, wobei die Ungesättigtheit nicht an dem dem Stickstoffatom benachbarten C-Atom vorliegt,
- ggfs. substituierte araliphatische Gruppen
- ggfs. substituierte aromatische Gruppen oder
- zusammen mit dem Stickstoffatom, an dem sie gebunden sind, sowie der am gleichen Stickstoffatom gebundenen anderen Gruppe einen gesättigten oder ungesättigten und ggfs. substituierten Heterocyclus, wobei sich die Ungesättigtheit nicht an dem dem Stickstoffatom benachbarten C-Atom befindet, oder
- zusammen mit dem Stickstoffatom, an dem sie gebunden sind, dem zentralen C-Atom, einem anderen Stickstoffatom und einer an diesem Stickstoffatom gebundenen Gruppe einen gesättigten oder ungesättigten und ggfs. substituierten Heterocyclus, wobei sich die Ungesättigtheit nicht an einem diesen Stickstoffatomen benachbarten C-Atom befindet,

$X_1$ $Cl^{\ominus}$, $Br^{\ominus}$, $I^{\ominus}$, $BF_4^{\ominus}$, $CN^{\ominus}$, $ClO_4^{\ominus}$, $NO_3^{\ominus}$, $NO_2^{\ominus}$, $OCN^{\ominus}$, $CH_3SO_4^{\ominus}$ oder $HSO_4^{\ominus}$ und
$X_2$ und $X_3$ gleichzeitig oder unabhängig Chlor oder Brom.

2. Verfahren nach Anspruch 1, dadurch gekennzeichnet, daß die Gruppen $R_1$ bis $R_6$ bedeuten:
Gesättigte aliphatische Gruppen mit 1 bis 12 C-Atomen oder ungesättigte aliphatische Gruppen mit 3 bis 12 C-Atomen, gesättigte oder ungesättigte cycloaliphatische Gruppen mit 5 bis 6 C-Atomen, araliphatische Gruppen mit 7 bis 12 C-Atomen oder Phenylgruppen oder zusammen mit der am gleichen Stickstoffatom gebundenen Gruppe und diesem Stickstoffatom einen fünf- bis sechsgliedrigen Heterocyclus, der ein weiteres Heteroatom enthalten kann, oder zusammen mit dem an einem anderen Stickstoffatom und der Gruppierung –N–C–N– einen fünf- oder sechsgliedrigen Heterocyclus.

3. Verfahren nach einem der vorhergehenden Ansprüche, dadurch gekennzeichnet, daß die Substituenten $R_1$ bis $R_6$ bedeuten:
Gesättigte aliphatische Gruppen mit 1 bis 4 C-Atomen oder ungesättigte aliphatische Gruppen mit 3 bis 8 C-Atomen, cycloaliphatische Gruppen mit 5 bis 6 C-Atomen oder Benzyl.

4. Verfahren nach einem der vorhergehenden Ansprüche, dadurch gekennzeichnet, daß die Substituenten der Gruppen $R_1$ bis $R_6$ unter Halogenatomen, Alkylgruppen, Alkoxygruppen, Aryloxygruppen und Nitrogruppen ausgewählt sind.

5. Verfahren nach einem der vorhergehenden Ansprüche, dadurch gekennzeichnet, daß $X_1$ ein Chloratom oder Bromatom und $X_2$ und $X_3$ Chloratome bedeuten.

6. Verfahren nach einem der vorhergehenden Ansprüche, dadurch gekennzeichnet, daß der Katalysator in einer Menge von $10^{-4}$ bis $5 \cdot 10^{-3}$ Äquivalent pro Äquivalent der Säure und vorzugsweise in einer Menge von $10^{-4}$ bis $10^{-3}$ Äquivalent pro Äquivalent der Säure eingesetzt wird.

7. Verfahren nach einem der vorhergehenden Ansprüche, dadurch gekennzeichnet, daß ein Phosgenüberschuß von 5 bis 25% angewandt wird.

8. Verfahren nach einem der vorhergehenden Ansprüche, dadurch gekennzeichnet, daß die Reaktionstemperatur im Bereich von 80 bis 160°C und vorzugsweise von 100 bis 140°C liegt.

9. Verfahren nach einem der vorhergehenden Ansprüche, dadurch gekennzeichnet, daß die Reaktion in einem Lösungsmittel oder einem Gemisch von Lösungsmitteln durchgeführt wird, die gegenüber Phosgen inert und unter aliphatischen chlorierten Kohlenwasserstoffen und aromatischen Kohlenwasserstoffen ausgewählt sind.

10. Verfahren nach einem der vorhergehenden Ansprüche, dadurch gekennzeichnet, daß die Carbonsäure eine Säure der Formel $R(COOH)_n$ ist, in der bedeuten:

R – eine geradkettige oder verzweigte, gesättigte oder ungesättigte und ggfs. substituierte aliphatische Gruppe mit bis zu 30 C-Atomen,
– eine ggfs. substituierte cycloaliphatische Gruppe mit 3 bis 6 C-Atomen,
– eine ggfs. substituierte araliphatische Gruppe,
– eine ggfs. substituierte aromatische Gruppe oder
– eine heterocyclische Gruppe

und

n 1, 2 oder 3.

11. Verfahren nach einem der vorhergehenden Ansprüche, dadurch gekennzeichnet, daß die Substituenten der Gruppe R unter Halogenatomen, Alkylgruppen, Halogenalkylgruppen, Alkoxygruppen, Phenoxygruppen und Nitrogruppen ausgewählt sind.

12. Verfahren nach einem der vorhergehenden Ansprüche, dadurch gekennzeichnet, daß es kontinuierlich durch gleichzeitige Einführung der Säure und des Phosgens durchgeführt wird.

13. Verfahren zur Herstellung der Komplexe der Formel II, in der $X_2$ und $X_3$ Chloratome bedeuten, dadurch gekennzeichnet, daß die durch Umsetzung von Chlorformamidiniumchlorid mit einem sekundären Amin erhaltenen Reaktionsprodukte mit Phosgen umgesetzt werden.